# EUROPEAN PATENT APPLICATION

(11) **EP 1 882 463 A1**
(43) Date of publication of application: **30.01.2008**
(21) Application number: 07252822.7
(22) Date of filing: 17.07.2007
(51) Int. Cl.: A61F 2/16

(54) **A light adjustable lens (LAL) allowing an improved retinal safety**

(30) Priority: 26.07.2006 US 493358
(71) Applicant: Calhoun Vision Inc., Pasadena, CA 91107 (US)
(72) Inventor: Gerlach, Mario, Altenberga 07768 (DE); Sandstedt, Christian A., Pasadena, CA 91101 (US); Chang, Shiao H., Arcadia, CA 91006 (US)
(74) Representative: Jackson, Richard Eric

(57) **Abstract**

Embodiments of the invention involve intraocular lenses that are light adjustable to change a focal length of the lens after it has been implanted into a person or animal. The lens includes a layer of UV absorbent material located on a rear side, which is the side facing a retina. The lens may comprise a UV reducing rim that surrounds a peripheral portion of the lens, and reduces or blocks UV light from striking the retina.

## Description

### CROSS-REFERENCE TO RELATED APPLICATIONS

This application is related to co-pending and commonly assigned U.S. Patent Application Serial Number 10/319,082, filed December 13, 2002, and published as 2003/0176521, on September 18, 2003, entitled "INITIATOR AND ULTRAVIOLET ABSORBER FOR CHANGING LENS POWER BY ULTRAVIOLET LIGHT," and U.S. Patent Application Serial Number 60/715,310, filed September 8, 2005, entitled "NOVEL ADJUSTABLE OPTICAL ELEMENTS WITH ENHANCED UV PROTECTION," the disclosures of which are hereby incorporated herein by reference.

### TECHNICAL FIELD

This application relates in general to intraocular lenses, and in specific to intraocular lenses that are capable of post-operative modification of their optical properties and to intraocular lenses that can be modified in-vivo by exposing them to radiation.

### BACKGROUND OF THE INVENTION

Cataract extraction followed by intraocular lens (IOL) implantation is the most commonly performed surgery in patients over 65 years old. See, Learning, D.V., "Practice Styles and Preferences of ASCRS Members-1999 Survey," J. Cataract Refract. Surg. 2000; 26(6); 913-21.

The power of the IOL lens can be estimated prior to implantation. However, errors in estimating the required power, as well as shifting of the lens post-operatively due to wound healing, often results in less than optimal vision. Consequently, a patient may have to choose between additional surgery to replace or reposition the lens or to use additional corrective lenses, e.g. spectacles.

### BRIEF SUMMARY OF THE INVENTION

Embodiments of the invention involve intraocular lenses that are capable of having their refractive power modified in-vivo by irradiation with the appropriate dose, spatial irradiance profile, and frequency of light. This novel type of intraocular lens is known as the light adjustable lens (LAL). The LAL consists of four basic components. The first is the matrix polymer, which gives the LAL its basic optical and mechanical properties. Homogeneously distributed throughout the matrix polymer is a chemical moiety referred to as the macromer. The macromer contains photopolymerizable endgroups that are capable of forming crosslinks between each other if it is exposed to light of the appropriate frequency. The third major component is the photoinitiator, which initiates the macromer crosslinking by forming highly reactive radicals after absorbing light of the appropriate frequency. The fourth major component is the UV absorber which acts to protect the retina from UV light. More extensive details regarding the chemical composition and mechanism for refractive power change can be found in the literature (U.S. patents 6,560,642, 6,721,043, 6,749,632, and 6,813,097, incorporated herein by reference). The typical wavelength(s) of light that is used to adjust the refractive power of the LAL is in the near-UV (e.g. 365 nm). After the LAL has been adjusted to the desired refraction it is typically necessary to perform a final photolocking step which involves irradiating the entire lens which consumes substantially all the remaining macromer. During photolocking some UV light may unintentionally spill over the outside edges of the LAL due to normal eye movement, variation in anterior chamber distance (ACD) of the LAL (i.e. magnificiation differences), and a dimensional difference in the lens from the manufactured process. Due to the potentially hazardous effects of UV light to the eye, particularly the retina, it is desirable to find solutions that permit the LAL to be locked-in while at the same time minimizing the amount of light striking the retina.

To prevent or reduce the amount of UV light striking the retina, the LAL, according to embodiments of the invention, includes a layer of UV absorbing material located on its posterior surface, i.e. the side facing the retina. Other embodiments include having the lens comprise a UV reducing rim that surrounds the peripheral portion of the lens, and reduces or blocks UV light from striking the retina. The rim may comprise a material that absorbs, reflects, and/or scatters the UV light. The rim may also comprise a structure such as frosting or a grating that scatters or diffracts the UV light.

One feature of an embodiment of the invention is to significantly enhance UV safety for patients using light adjustable intraocular lens.

Another feature of an embodiment of the invention is that the UV layer and/or the UV rim do not require in-vivo lock-in.

A further feature of an embodiment of the invention is that the UV layer and/or the UV rim do not act as a diffusion reservoir of unpolymerized macromers, which would interfere with the light adjustable procedure.

A still further feature of an embodiment of the invention is that the UV layer and/or the UV rim assists in the prevention of cells from migrating to the back of the LAL, which leads to an improved PCO rate. The PCO rate can be significantly reduced using sharp-edged lens designs that involve certain pressure to the posterior capsule. The additional UV safety rim may be advantageously shaped in the form of a sharp edge design of particular angulations.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawings, in which:

FIGURE 1 depicts an embodiment of the light adjustable lens being irradiated to form a portion with increased power;

FIGURES 2A-2C depict an example of a lens according to embodiments of the invention;

FIGURE 3 is a graph depicts retinal dose versus lens absorption, according to embodiments of the invention;

FIGURE 4 depicts light spill over from a lens that lacks a rim, according to embodiments of the invention;

FIGURES 5A-5C depict an example of a lens according to embodiments of the invention;

FIGURES 6A-6B depict examples of rims for a lens according to embodiments of the invention;

FIGURE 7 depicts a lens according to embodiments of the invention;

FIGURES 8A-8D depict different arrangements for a rim according to embodiments of the invention;

FIGURES 9A and 9B depict different arrangements for a rim according to embodiments of the invention involving phase gratings located within the rim; and

FIGURES 10A and 10B depict other embodiments of the invention involving a UV blocking area that is associated with the haptics of a lens.

### DETAILED DESCRIPTION OF THE INVENTION

A light adjustable lens (LAL) comprises four distinct chemical entities or components. The first component is the matrix polymer that gives the lens its basic shape, refractive index, and material properties. The second component is known as the macromer and is, by design, chemically similar to the matrix polymer. One difference between the two is the presence of photopolymerizable end groups on the macromer such that the application of the appropriate frequency of light will cause the macromer molecules to form chemical bonds between each other. The third component is the photoinitiator that absorbs the applied incoming light (365 nm) and initiates polymerization of the macromer. The fourth component of the LAL is a UV absorbing molecule that protects that retina from ambient UV irradiation.

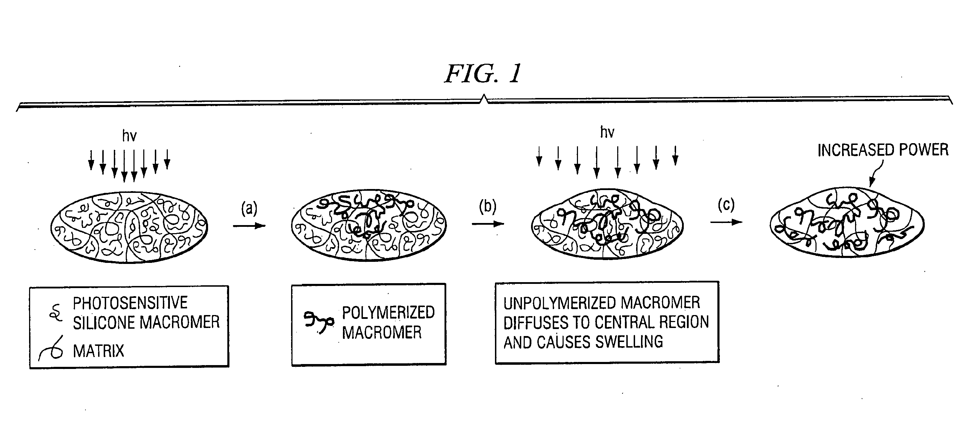

The mechanism upon which the LAL technology is based is depicted graphically in FIGURE 1. Application of light to the LAL will cause the photopolymerized macromers in the irradiated region to form an interpenetrating network within the target area of the lens. This action produces a change in the chemical potential between the irradiated and unirradiated regions of the lens. To reestablish thermodynamic equilibrium, macromers in the unirradiated portion of the lens will diffuse into the irradiated region producing a swelling in the irradiated region that effects a change in the lens curvature. As an example, if the central portion of the lens is irradiated and the outside portion is left nonirradiated, unreacted macromer diffuses into the center portion causing an increase in the lens power and a hyperopic shift. Likewise, by irradiating the outer periphery of the lens, macromer migrates outward causing a decrease in the lens power and a myopic correction. By controlling the irradiation dosage (i.e., beam intensity and duration), spatial intensity profile, and target area, physical changes in the radius of curvature of the lens surface are achieved, thus modifying the refractive power of an implanted LAL to either add or subtract spherical power, adjust along toric axes, or correct higher order aberrations. Note that multiple zones may be irradiated to form a multi-focus lens, e.g. a bulls eye lens. Once the appropriate power adjustment is achieved, the entire lens is irradiated to polymerize the remaining unreacted macromer to prevent any additional change in lens power. By irradiating the entire lens, macromer diffusion is prevented, thus no change in lens power results. This second irradiation procedure is referred to as "lock-in".

The LAL of FIGURE 1 requires the application of UV-A light (i.e. 365 nm) to initiate the photopolymerization process. The natural human optical media are transmissive in the UV-A wavelength range. According to published results and experiments performed at Calhoun Vision, Inc. (Pasadena, CA) the natural human cornea transmits around 50% of the incident light at 365 nm. In addition, measurements of human aqueous and vitreous indicate that greater then 90% of the light at 365 nm is transmitted, so that some of the 365 nm light once inside the eye will reach the retina. UV-A is considered to cause negative photochemical effects to the human retina like Dyschromatopsia ("Blue cone knock out"). In order to avoid phototoxic effects, several international standardization committees (e.g. ICNIRP) have defined wavelength specific maximum permissible retinal exposure values (MPE). Clinical trials using the LAL technology indicate that even at doses significantly below the given limit values, patients perceive disturbed color vision for a couple of days post irradiation.

Due to the fact that the lens body is homogeneously formed of photosensitive material, a comprehensive lock-in of the entire lens is required to stabilize its refractive properties. Failure to do so, may result in unintended power changes by exposing the eye to ambient source of UV, e.g. sunlight. The requirement that the LAL is locked-in up to the outer edges causes the major disadvantage with regard to UV safety.

In theory, the lock-in intensity pattern matches exactly the diameter of the LAL. Therefore, the entire beam is refracted into the LAL and absorbed within the material according to Lambert-Beers law. However, a minor, nonabsorbed portion of the beam is still transmitted trough the LAL and forms a resulting intensity pattern at the retina.

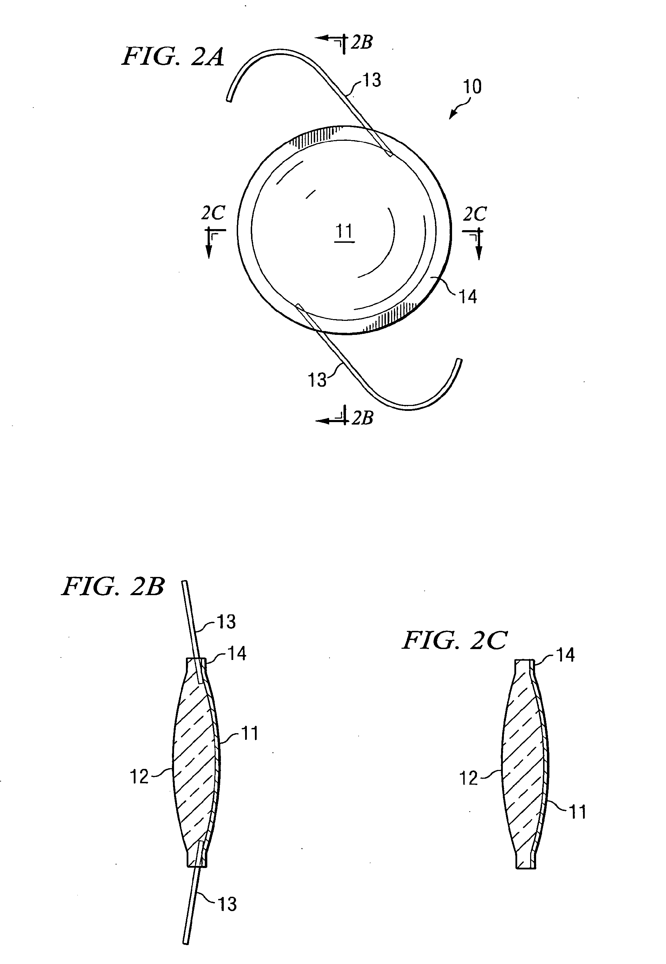

One embodiment of the invention reduces the amount of UVA light striking the retina. This embodiment involves a new lens molding technique that allows a thin (50-100 µm), highly absorbing UV layer to be attached to the posterior surface of the LAL. An example of such a lens is shown in FIGURES 2A-2C. FIGURE 2A depicts a rear elevational view of lens 10, where the rear side of the lens includes a UV absorbing layer 11. A particularly useful class of UV-absorbing compounds that comprise the UV absorbing layer is selected from compounds having the following formula or structure: 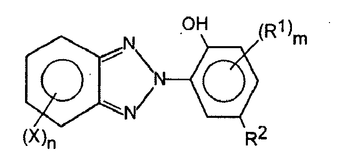 wherein each x is independently selected from the group consisting of H, halogen, alkyl, hydroxyl, amino, carboxyl; each R¹ is independently selected from the group consisting ofH, alkyl, substituted alkyl, alkoxy, substituted alkoxy, hydroxyl, amino and carboxyl; R² contains a vinyl moiety; m is an integer from 1-3; and n is an integer from 1-4. The preferred UV-absorbing compounds absorb UV light strongly in the range 300 nm to 400 nm, and exhibit reduced absorption at wavelengths higher than about 400 nm. The amount of UV absorber required to give the desired amount absorption is dependent upon the specific UV absorber, its concentration in the layer, and its optical path length. The lens 10 comprises a light adjustable region 12, and haptics 13. FIGURE 2B depicts a sectional view of lens 10 of FIGURE 2A along line A-A. FIGURE 2C depicts a sectional view of lens 10 of FIGURE 2A along line B-B.

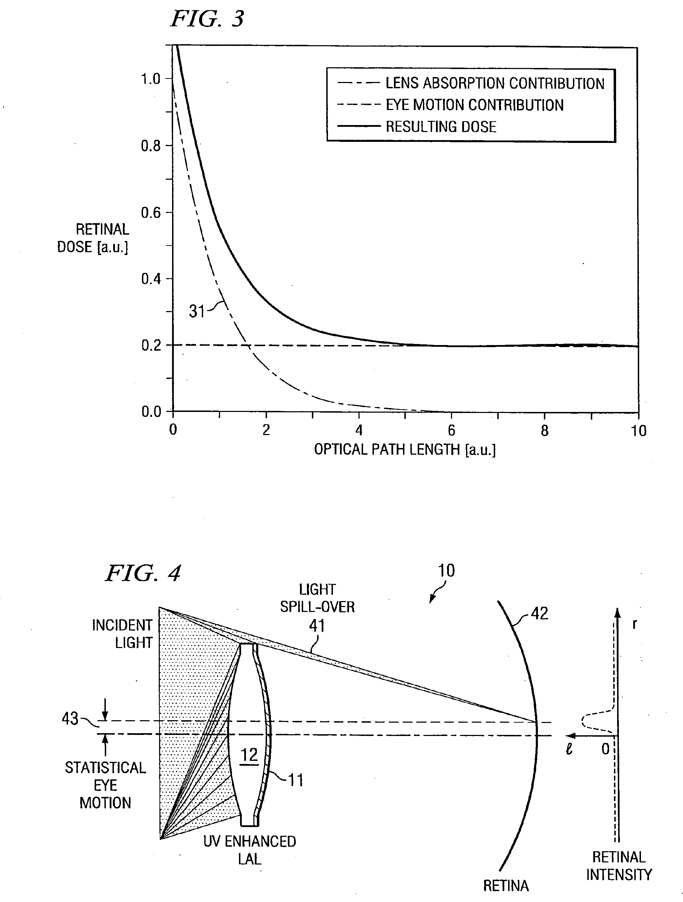

The majority of the UV radiation applied to the LAL during the lock-in process is absorbed by the lens lock-in process. The remaining fraction of the incident radiation depends on the absorption coefficient of the material of the bulk lens portion 12 and absorbing layer 11, as well as the individually traveled optical path length. FIGURE 3 depicts a graph of the retinal dose versus the lens absorption. Line 31 illustrates the dependency of the retinal dose on the optical path length of the lens. As expected, increasing the optical path length of the lens causes the retinal dose to decrease exponentially. The reduction in light at the retina is accomplished by increasing the LAL thickness, e.g. portion 12 and/or increasing the backing layer thickness, e.g. layer 11. The remaining retinal intensity can be theoretically decreased by several magnitudes by this approach, as the convergence of line 31 of FIGURE 3 indicates.

The previous discussion considered an idealized case where the lock-in beam is the same size as the LAL (e.g. 6 mm), the beam is focused in the central volume of the LAL, and that the irradiating beam is aligned exactly to the LAL in the x and y planes. However, in clinical practice the above assumptions are not necessarily valid due to diameter manufacturing tolerances of individual LALs and variation in patient anterior chamber depth (ACD) that cause slight variation in the magnification of the lock-in beam at the LAL. These two factors can conspire together to make the LAL smaller than the incident beam, which will permit rays at the edge of the LAL to pass unattenuated to the retina as illustrated in FIGURE 4. In FIGURE 4, spill over light 41 from the lock-in process passes to the retina 42.

Also illustrated in FIGURE 4 is another factor that contributes to unattenuated light passing around the LAL to the retina, which is a patient's natural saccadic eye motion 43 during the treatment procedure. This motion will cause both temporal defocus and decentration of the beam at the LAL. The magnitude of the temporal spillover depends on the patient/physician individual motion statistics. Thus, increasing the thickness or increasing the absorption coefficient of the UV absorbing back layer to decrease the dose at the retina may not be sufficient to drop the dose at the retina below levels that will produce erythropsia in a majority of the patients.

Another embodiment of the invention is an UV blocking safety rim. This rim significantly enhances UV safety for patients, and does not require in-vivo lock-in. The rim also does not act as a diffusion reservoir of unpolymerized macromers. The rim further prevents cells from migrating to the back of the LAL, leading to an improved PCO rate. The PCO rate can be significantly reduced using sharp-edged lens designs that involve certain pressure to the posterior capsule. The additional UV safety rim may be advantageously shaped in the form of a sharp edge design of particular angulations.

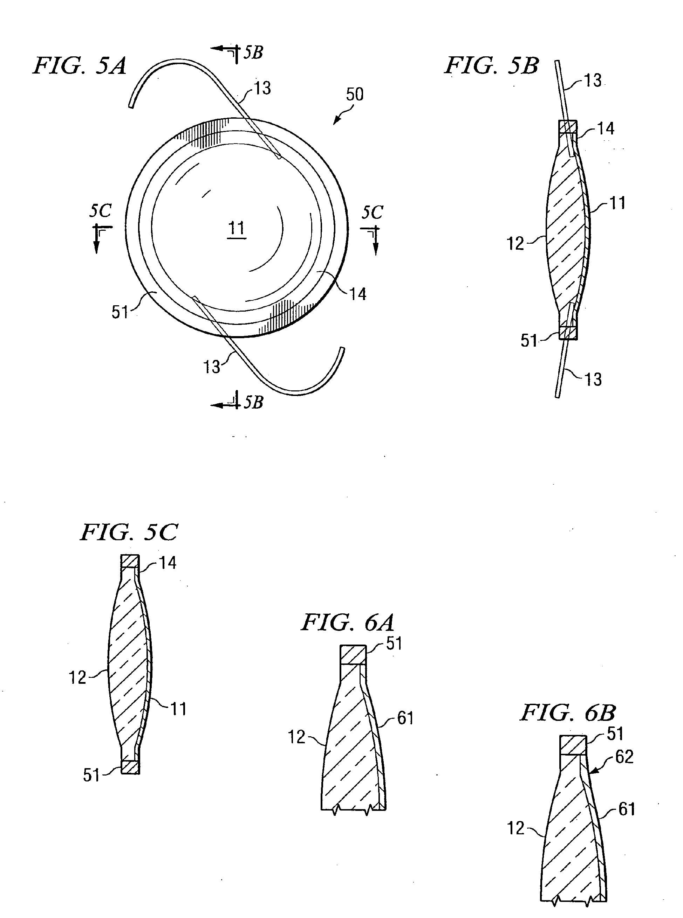

Another embodiment of the invention involves using a UV-light blocking rim around the LAL. An example of this embodiment is shown in FIGURE 5A. This example lens 50 includes rim 51, will reduce or prevent light spill over from eye movements and/or lens size variation. FIGURE 5B depicts a sectional view of lens 50 of FIGURE 5A along line A-A. FIGURE 5C depicts a sectional view of lens 50 of FIGURE 5A along line B-B.

To determine a diameter of this UV blocking rim, the amplitude of eye motion was measured for a series of patients with an implanted LAL during their respective treatment procedures. A statistical analysis of this motion data indicates that 90% of all patient lens motion was within 230 µm of alignment. Therefore, designing an outer UV absorbing rim that has a radius of 250 µm on each side should reduce greater than 90% of the spill over contribution.

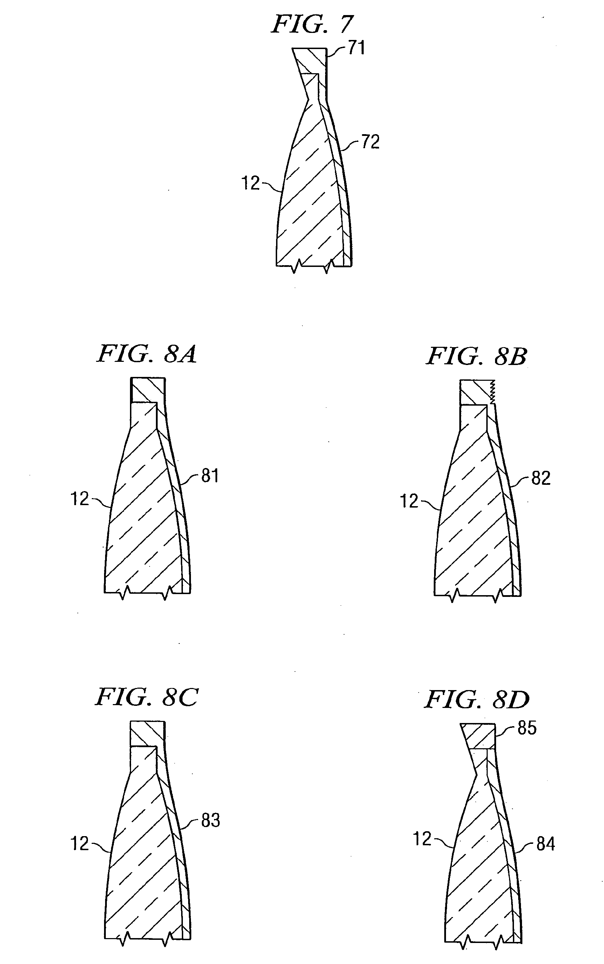

One embodiment of the UV blocking rim may have the rim material be transparent, and include a layer of material to the front side and/or back side of the rim, which is fully opaque in UV light and visible light, for example a black or colour pigmented layer as in lens 81 of FIGURE 8A. Another embodiment of the UV blocking rim may have the rim material be transparent in UV and visible light, and include surface modifications, such as frosting, blazed/nonblazed diffractive structures that direct the light outward in the peripheral retina, prisms, nonblazed wedge structures or other types of blazed wedge structures to the front side and/or back side of the rim, for example lens 82 of FIGURE 8B.

These modifications refract or scatter the unwanted light from the light path. A further embodiment of the UV blocking rim may have the rim material be clear in visible light and be highly absorbing in UV light, for example lens 83 of FIGURE 8C. One type of material is the same material used as the UV layer 11. The rim may be photo-locked prior to implantation or as part of the manufacturing cycle. A still further embodiment may have the rim comprise opaque material the blocks both UV and visible light, such as lens 84 of FIGURE 8D. The different edge designs of FIGURES 8A-8D avoid or minimize visible stray light and glare at large pupil dilations.

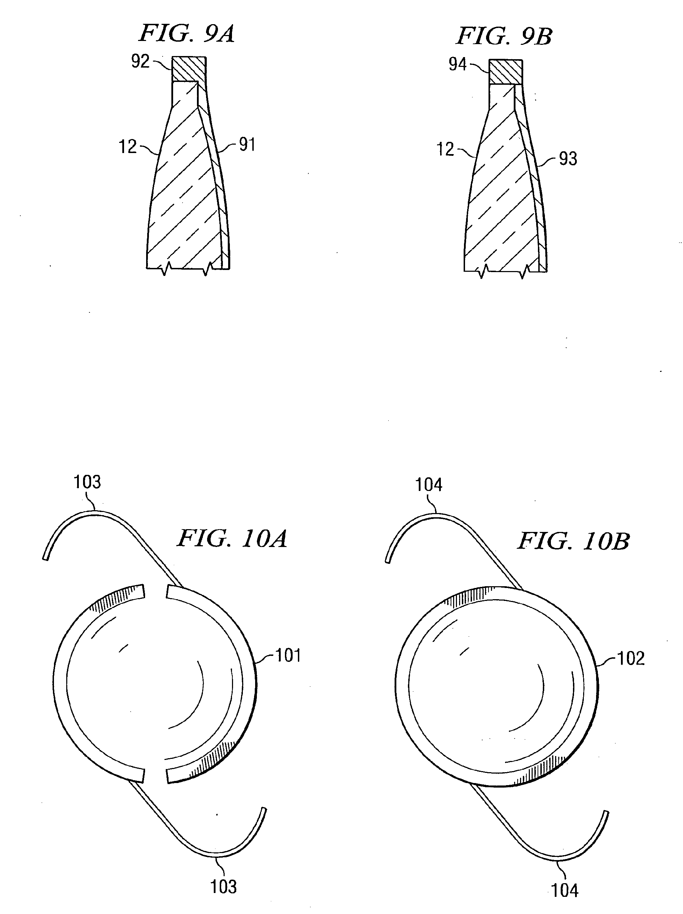

Furthermore, another embodiment may involve modifications in the refractive index such as creating gradient index optical surface or regular phase gratings in the material portion of the rim, for example as shown in FIGURES 9A and 9B. In FIGURE 9A, the rim portion 92 with an internal phase grating is integral with the backing layer 91. In this embodiment, rim portion 92 may comprise the same material as layer 91. In FIGURE 9B, the rim portion 92 with an internal phase grating is separate from the backing layer 91. In this embodiment, rim portion 92 may comprise the same material as layer 91 or a different material. The implementation of the refractive index gradients is arranged in such a way as to direct the majority of radiation into the peripheral retina (well outside the macula) while also avoiding the creation of regions of peak irradiance in these peripheral regions.

Another embodiment of the invention is an UV blocking safety rim that is not limited to the IOL material or its derivatives and is accomplished by creating particularly shaped UV-blocking haptics and/or haptic joints, for example as shown in FIGURES 10A and 10B. In FIGURE 10A, the dual piece haptics 103 include the UV blocking area 101. In FIGURE 10B, the single piece haptics 104 include the UV blocking area 102.

Lens 50 of FIGURE 5A may be produced by prelocking of the UV-backing layer during manufacturing process. The backing layer could be entirely locked in after molding. The bulk will be molded onto the inactive backing in a second step. Moderate heat will be applied afterwards to allow thermal healing of the interface. The fully polymerized backing layer could potentially prevent diffusion of macromers into it.

FIGURES 6A and 6B depict alternative embodiments of the lens. In FIGURE 6A, the UV layer 61 is substantially uniform in thickness. In FIGURE 6B, the UV layer 62 varies in thickness, for example layer 62 may be thicker where layer 12 is thinner. This will even out the UV blocking of the layers 12 and 62.

FIGURE 7 depicts another embodiment of the invention. In FIGURES 5A-5C, and 6A-6B, rim 51 is separate from layer 11. In FIGURE 7, rim portion 71 is integral with layer 72. In this embodiment, rim portion 71 may comprise the same material as layer 72.

Note that the haptics shown in various embodiments are not limited to c-loop configuration, as other types may be used.

A blocking layer may be positioned between layer 11 and layer 12 and/or between layer 12 and rim 51 to ensure that no unpolymerized macromer can diffuse in or out of the layer 12 and/or rim 51. Alternatively, materials may be chosen for the different layers whose chemical structure prevents the macromer from diffusion (i.e. steric hindrance).

Note that the lens shape may be flattened in the annular area 14 in order to provide sufficient edge thickness for joining the bulk lens with the haptics. The transition from the flattened area to the lens body can be shaped differently. FIGURE 5A includes a sharp edge resulting from basic joining of the geometries; however FIGURE 6B discloses a smooth transition with a radius r_{T} >> 0. This configuration helps to reduce the risk of PCO (posterior capsular opacification) by forming a tight contact interface between the posterior capsule which prevents epithelial cells from migrating onto the posterior lens surface.

Another embodiment has the layer 11 and rim 51 comprised of the same photo-active material. This material has high UV-blocking capabilities that is accomplished either through absorption of optical radiation or reflective properties. This layer 11 is extended towards the edges to form the safety rim 51. The use of the same material composition as used in the backing layer requires that the rim also needs to be photolocked since this rim will act as a reservoir of unpolymerized macromers which may cause unintended power changes in the future.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. An intraocular lens comprising:
a first portion that includes a material that is optically reactive to an external stimulus to change an optical characteristic of the lens; and
an ultra-violet (UV) light reducing layer that is adjacent to the first portion and absorbs a portion of UV light that is incident onto the layer.

2. The lens of claim 1, further comprising:
an annular rim that is located adjacent to a peripheral portion of the lens, wherein the rim comprises an UV light reducing structure.

3. The lens of claim 2, wherein the UV light reducing structure is located on a side of the lens that would face a retina.

4. The lens of claim 2, wherein the UV light reducing structure is located on a side of the lens that would face away from a retina.

5. The lens of claim 2, wherein the UV light reducing structure is located inside the rim.

6. The lens of claim 2, wherein the UV light reducing structure is a layer of material that is opaque to UV light.

7. The lens of claim 2, wherein the UV light reducing structure is a layer of material that is opaque to UV light and visible light.

8. The lens of claim 2, wherein the UV light reducing structure is a layer of material that absorbs UV light.

9. The lens of claim 2, wherein the UV light reducing structure comprises material that absorbs UV light.

10. The lens of claim 2, wherein the UV light reducing structure is a layer of material that is blocks a portion of the UV light.

11. The lens of claim 2, wherein the UV light reducing structure is a layer of material that has a roughed texture that scatters UV light.

12. The lens of claim 2, wherein the UV light reducing structure is a layer of material that reflects to UV light.

13. The lens of claim 2, wherein the UV light reducing structure is a layer of material that is opaque to UV light.

14. The lens of claim 2, wherein the UV light reducing structure is implemented as a surface grating that diffracts the light entering this portion of the intraocular lens.

15. The lens of claim 2, wherein the UV light reducing structure is formed by an implemented phase grating of varying refractive index that diffracts the light entering this portion of the intraocular lens.

16. The lens of claim 2, wherein the rim comprises: 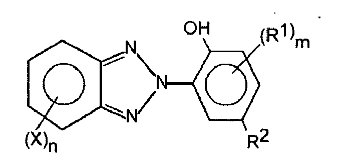 wherein each x is independently selected from the group consisting of H, halogen, alkyl, hydroxyl, amino, carboxyl; each R¹ is independently selected from the group consisting of H, alkyl, substituted alkyl, alkoxy, substituted alkoxy, hydroxyl, amino and carboxyl; R² contains a vinyl moiety; m is an integer from 1-3; and n is an integer from 1-4.

17. The lens of claim 1, wherein the optical characteristic is focal length.

18. The lens of claim 1, wherein the optical characteristic is the radial refractive power distribution along arbitrary meridians.

19. The lens of claim 1, wherein the optical characteristic is an arbitrary local refractive power distribution.

20. The lens of claim 1, wherein the change occurs by photopolymerization.

21. The lens of claim 1, wherein the external stimulus is UV light.

22. The lens of claim 1, wherein the layer is located on a side of the lens that would face a retina.

23. The lens of claim 1, further comprising a particularly shaped UV-blocking haptic rim.
